# EUROPEAN PATENT APPLICATION

(11) **EP 3 640 333 A1**
(43) Date of publication of application: **22.04.2020**
(21) Application number: 18832264.8
(22) Date of filing: 25.06.2018
(51) Int. Cl.: C12N 15/85, C12N 5/10

(54) **GENE EDITING SYSTEM AND GENE EDITING METHOD**

(30) Priority: 14.07.2017 CN 201710574537
(71) Applicant: Cure Genetics Co., Ltd, Suzhou, Jiangsu 215123 (CN)
(72) Inventor: LIN, Yanni, Suzhou Jiangsu 215123 (CN); XU, Yuanyuan, Suzhou Jiangsu 215123 (CN); LI, Jun, Suzhou Jiangsu 215123 (CN)
(74) Representative: Berger, Axel Bernhard
(86) International application number: PCT/CN2018/092710
(87) International publication number: WO 2019/011118

(57) **Abstract**

The present invention relates to a CRISPR/Cas system comprising guide RNAs and a Cas protein, wherein the guide RNAs are composed of the following two parts: the guide RNAs are capable of respectively binding to two sequences of target sequences having SEQ ID NO: 2, SEQ ID NO: 3 and SEQ ID NO: 4 in eukaryotic B2M genes. The system exhibits a relatively high single-gene knockout rate for B2M. The present invention further relates to a gene editing method for T cells using the system, which method provides an efficient, safe and simple process for producing universal T cells.

## Description

### Technical Field

The present invention relates to a gene editing system and a gene editing method, in particular to a CRISPR-Cas system, and a gene editing method for T cells using the system.

### Background Art

Recently, it has been gradually proven that autologous immune cell reinfusion (also known as adoptive immunotherapy) has a strong therapeutic effect in the treatment of chronic infections or cancers. In the T cell-based adoptive immunotherapy, tumor antigen-specific T cells (e.g., tumor infiltrating lymphocytes (TIL)), and T cells that have been genetically modified to express artificial receptors (e.g., chimeric antigen receptors (CAR)) or natural receptors (T cell receptor (TCR)) are usually used to direct T cells to attack cancer cells (Johnson et al. Cell Research 2017. 27: 38-58). The CAR molecule is composed of a single-chain antibody variable fragment (scFv), a suitable signaling domain, and a costimulatory domain. T cells with the CAR are activated when the antigen of tumor cells is recognized by the scFv of the CAR molecule (Sadelain et al. Cancer Discov 2013. 3: 388-398). Compared to TCR, CAR molecules are not restricted by human leukocyte antigen (HLA) molecules, and therefore, CAR is suitable for patients with any HLA match. The chimeric antigen receptor T cell (CAR-T) therapy has been successfully applied in several clinical trials for hematological malignancies, and many researchers have been actively testing the application thereof in the treatment of different solid tumors (Johnson et al. Cell Research 2017. 27: 38-58).

The autologous immune cell therapy requires the use of the patient's own immune cells, and thus creates many challenges in terms of technology, production, logistics, etc. Patients who are suitable for the adoptive immunotherapy usually have received multiple rounds of chemotherapy or radiotherapy, which impairs the immunocyte function of these patients, so it is very difficult to culture and expand these cells. Moreover, the autologous immune cell therapy requires individualized and customized cell preparations, and separate pipelines, spaces and disposable containers during the production process for each patient, resulting in an extremely high cost for the autologous immune cell therapy. Furthermore, for the autologous immune cell therapy, T cells isolated and collected from a patient *in vivo* are required to be transported to a production workshop of cell preparations for processing, and then transported back to the hospital for reinfusion into the patient. The combined time periods of the round-trip transportation and production process are more than a month, during which the patient's condition continues to deteriorate, thus missing the optimal opportunity for treatment. These shortcomings of the autologous immune cell therapy further limit its wide application.

The cell immunotherapy based on allogeneic immune cells has the potential to provide "ready-to-use" treatment regimens for patients, thereby solving the problems existing in autologous immune cell therapy as mentioned above. Allogeneic T cells obtained from the donor can recognize the allogeneic antigen of the recipient patient via endogenously expressed TCR, and then attack the recipient patient, resulting in a fatal graft versus host reaction (GvHD). Meanwhile, the mismatch of HLA on the surface of allogeneic T cells can induce rapid clearance of the allogeneic T cells by the immune system of the recipient patient (i.e., a host versus graft reaction (HvGR)), thereby shortening the retention time of the reinfused cells in the recipient patient and reducing the therapeutic effect. Therefore, the preparation of "ready-to-use" T cells requires a mature technical approach to knock out TCR and HLA (i.e., human major histocompatibility complex (MHC)) related genes.

The clustered regularly interspaced short palindromic repeat (CRISPR) and CRISPR-binding protein (Cas9) form a powerful nuclease system capable of cleaving almost all genomic sequences adjacent to the protospacer-adjacent motif (PAM) in eukaryotic cells (Cong et al. Science 2013. 339: 819-823). In addition to the Cas9 protein, the RNA component contained in the CRISPR/Cas9 system has a double-stranded guide RNA structure consisting of a CRISPR RNA (crRNA) and a trans-activated crRNA (tracrRNA), which directs the Cas9 protein to cleave DNA sites complementary to the crRNA sequence (Jinek et al. Science 2012. 337: 816-821). To further simplify the CRISPR/Cas9 system, researchers used engineering methods to link the two components (crRNA and tracrRNA) of the double-stranded guide RNA to form a chimeric single-stranded guide RNA (sgRNA) (Jinek et al. Science 2012. 337: 816-821; Cong et al. Science 2013. 339: 819-823; Mali et al. Science 2013. 339: 823-826; Hsu et al. Nat Biotechnol 2013. 31: 827-832). When different genomic sites are targeted, it is only necessary to change the crRNA sequence (or the variable region of sgRNA of about 20 bases) according to the DNA sequence of the target site without changing the Cas9 protein and tracrRNA (or the constant region of sgRNA of about 80 bases). Therefore, simultaneous cleavage of multiple genomic sites can be achieved by adding different crRNAs or sgRNAs to the system. The multi-point cleavage capability of the CRISPR/Cas9 system is far superior to other gene editing systems such as the zinc finger nuclease (ZFN) system and the transcription activator-like effector nuclease (TALEN) system, which is because the DNA binding proteins in these two systems have to be remodified for each new targeted DNA sequence to achieve new targeted functions.

When delivered into cells, specific nucleases, such as CRISPR/Cas9, ZFN or TALEN, produce a DNA double-stranded break (DSB) at the target site on the genome. The intracellular DNA repairing mechanism can repair DSB by means of non-homologous end-joining (NHEJ), which can introduce random insertion or deletion, and destroy and knock out the target genes. When cells are provided with a repair template, DSB can be repaired by means of homologous directed repair (HDR), wherein the genomic sequence repaired by this means will be consistent with the template sequence, resulting in a change in the customized sequence at the target site, such as site-directed base mutations or insertion of exogenous gene fragments.

Patent application documents WO 2013/176915 and WO 2015/136001 disclose a technical solution for knocking out TCR and MHC class I molecule related genes using TALEN. Patent application document WO 2015/136001 also lists the gRNA (guide RNA) sequences that may target three exons of the gene encoding the TCR, but does not disclose any specific data regarding these gRNA-mediated gene mutation/knockout frequencies.

An article published in Clinical Cancer Research in 2016 reported that a combination of multiple sgRNAs and the mRNA encoding Cas9 were used to knock out the TCR gene and the beta-2 microglobulin gene (B2M), which is an important component of MHC class I molecules, thereby clearing the TCR and MHC class I proteins presented on the surface of T cells (Ren et al. Clinical Cancer Research 04 November 2016). However, in this report, the simultaneous delivery of Cas9 mRNA and sgRNA by an electroporation-transfection method can only obtain less than 10% TCR-negative T cells, and the only way to obtain a higher gene knockout efficiency is to perform a second electrotransfection the next day. However, the results show that the second electrotransfection on the next day will greatly affect the proliferation capability of T cells, resulting in a decrease in the expansion folds of the T cells. Moreover, an additional electrotransfection step will increase the complexity of the production process and the production cost per batch of cell preparations.

In order to increase the effective amount of sgRNA, researchers used a lentiviral vector to permanently insert a sequence expressing sgRNA into the genome of a T cell, thereby knocking out the TCR and B2M genes (Ren et al. Oncotarget 2017. 8:17002-17011). However, the stable and permanent expression of sgRNA in T cells can lead to unpredictable off-target effects, such as RNA interference effects, thereby increasing the uncontrollability and potential toxicity of the allogeneic cell therapy.

The purified Cas9 proteins can be pre-bound with *in vitro* transcribed or synthesized gRNAs to form a ribonucleoprotein (RNP) complex, and the complex exerts its function by transfecting same into cells. Since the half life of the RNP composed of proteins and RNA in cells is very short, it is safer to deliver the CRISPR/Cas9 system in this form into cells than that in the form of mRNA, plasmids, and viral vectors. In the previous reports, the Cas9 RNP method has a TCR single-gene knockout rate of about 37% (with a total range from 30% to 45%) on average, but a TCR/B2M double-gene knockout rate of only about 25% (with a total range from 20% to 30%) on average (Liu et al. Cell Research 2017. 27: 154-157). There is still much room for improvement in the efficiency of the RNP gene knockout.

### Summary of the Invention

The present invention describes a method using a CRISPR/Cas9 system, which method uses an undisclosed gRNA combination, thereby greatly improving the efficiency of gene editing for manufacturing universal T cells and efficiently achieving the single-gene knockout rate of TCR or B2M, and a TCR and B2M double-gene knockout rate of 55% on average on the premise that no enrichment means is used. When the chemically modified gRNA is used, where no enrichment is involved, the TCR single-gene knockout efficiency and TCR and B2M double-gene knockout efficiency can reach an unprecedented high efficiency respectively, i.e., more than 90% for the single-gene knockout efficiency and more than 80% for the double-gene knockout efficiency, respectively.

The present invention relates to a CRISPR-Cas system comprising guide RNAs and a Cas protein, wherein the guide RNAs are composed of the following two parts: a first guide RNA, capable of binding to a target sequence having SEQ ID NO: 1 in eukaryotic TRAC genes; and second and third guide RNAs, capable of respectively binding to two sequences of target sequences having SEQ ID NO: 2, SEQ ID NO: 3 and SEQ ID NO: 4 in eukaryotic B2M genes.

The present invention also relates to a method for preparing engineered eukaryotic cells, comprising transfecting the eukaryotic cells with the CRISPR-Cas system of the present invention, and selectively inactivating genes encoding TRAC and B2M.

The CRISPR-Cas system of the present invention can be used to produce universal T cells, enabling cells from an allogeneic donor to be transplanted into a patient to treat conditions that require cell therapies, such as cancer, infection, or immune disease.

The present invention further relates to a CRISPR-Cas system comprising guide RNAs and a Cas protein, wherein the guide RNAs are composed of the following two parts: the guide RNAs are capable of respectively binding to two sequences of target sequences having SEQ ID NO: 2, SEQ ID NO: 3 and SEQ ID NO: 4 in eukaryotic B2M genes; preferably binding to two sequences of the target sequences having SEQ ID NO: 2 and SEQ ID NO: 3, or two sequences of the target sequences having SEQ ID NO: 2 and SEQ ID NO: 4, or two sequences of the target sequences having SEQ ID NO: 3 and SEQ ID NO: 4. The system exhibits a relatively high single-gene knockout rate for B2M.

We also describe for the first time a method of gene editing by mixing lymphocytes from multiple unrelated donors. Since primary T cells are taken from the peripheral blood of human donors, the maximum number of cells that can be obtained from a single donor at a time is limited, and in the prior art, cells from different donors are required to be processed in batches, which cause a limit to the number of cells that can be produced in a single batch. For the first time, we successfully edited a mixture of cells from multiple donors so that cells produced in a single batch are not necessarily from the same donor, and therefore, the number of cells that can be processed in a single batch can be increased, thereby further reducing the production cost.

Our method provides an efficient, safe and simple process for producing universal T cells. These universal T cells do not induce problems of GvHD or HvGR and are therefore suitable for the cell therapy of allogeneic cell reinfusion.

### Brief Description of the Drawings

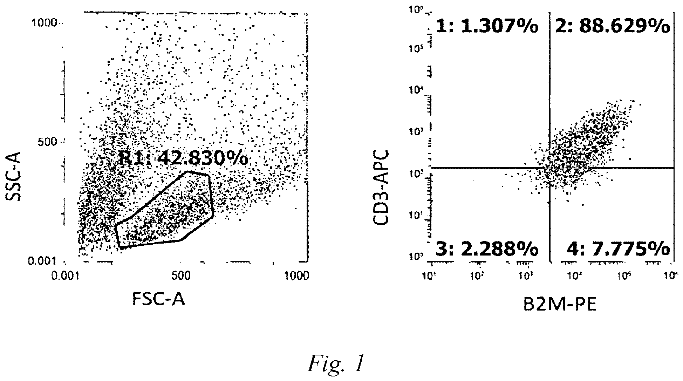

**Figure 1** shows the flow cytometry analysis of the presentation of CD3 and B2M on human primary lymphocytes after activation.

Samples are stained with antibodies on day 5 after transfection of control RNA (without CRISPR) by electroporation. In the left panel, FSC-A is the forward scatter-area, and SSC-A is the side-scatter area, wherein the R1 circled area in the panel indicates the lymphocyte population. In the right panel, CD3-APC represents the fluorescent signal of anti-CD3 antibody fluorescently labeled with APC; B2M-PE represents the fluorescent signal of anti-B2M antibody fluorescently labeled with PE; and the cells in the upper right quadrant are the double-positive cell populations stained with these two antibodies.

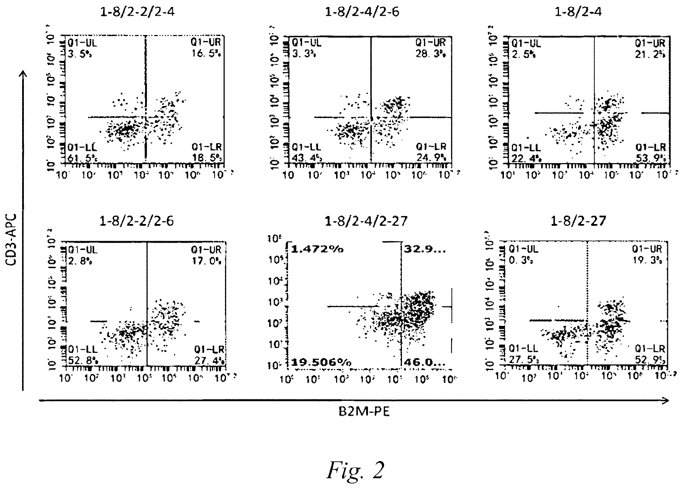

**Figure 2** shows the flow cytometry analysis of the efficiency of simultaneous knockout of TCR and MHC class I molecules in primary human T cells by different CRISPR/Cas9 guide RNA combinations.

Cas9 and the guide RNA combinations (each RNA is mixed in an equal mass ratio) shown above each panel of flow cytometry are delivered to primary cells and then tested for the double-gene knockout efficiency thereof. The horizontal axis represents B2M staining, and the vertical axis represents CD3 staining. The lower left quadrant of the panel of flow cytometry includes CD3-B2M-double negative cells, representing the TRAC and B2M double-gene knockout efficiency.

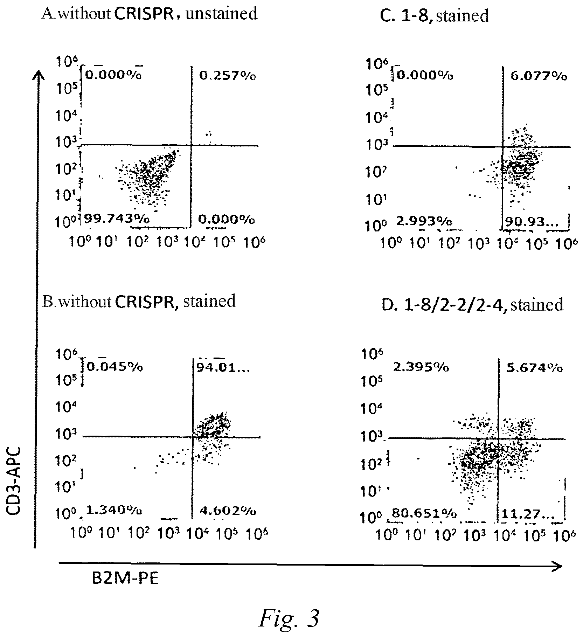

**Figure 3** shows the use of the chemically modified guide RNA to achieve efficient single- and double-gene knockouts in human primary T cells.

Panel (A): unstained control cells, without involving CRISPR. Panel (B): stained control cells, without involving CRISPR. Panel (C): stained cell samples, which are edited with 1-8 CRISPR/Cas9. Panel (D): stained cell samples, into which Cas9 and 3 guide RNAs, 1-8, 2-2 and 2-4, are delivered in a mass ratio of 2 : 1 : 1. Fluorescently-labeled CD3 and B2M antibodies are used for staining. The horizontal axis represents B2M staining, and the vertical axis represents CD3 staining. The lower left quadrant of the panel of flow cytometry includes CD3-B2M-double negative cells, representing the TRAC and B2M double-gene knockout efficiency.

### Detailed Description of The Invention

The present invention is described in detail herein with reference to the following definitions and embodiments. The content of all the patents and publication documents referred to herein, comprising all the sequences disclosed in these patents and publication documents, are expressly incorporated herein by reference.

### CRISPR/Cas System

The CRISPR/Cas system is a nuclease system formed by the clustered regularly interspaced short palindromic repeat (CRISPR) and CRISPR-binding protein (i.e., Cas protein), which system is capable of cleaving almost all genomic sequences adjacent to the protospacer-adjacent motifs (PAM) in eukaryotic cells (Cong et al. Science 2013. 339: 819-823). "CRISPR/Cas system" refers collectively to transcripts involved in CRISPR-associated ("Cas") genes, and other elements involved in the expression or directing the activity of CRISPR-associated genes, including sequences encoding a Cas gene, a tracr (trans-activated CRISPR) sequence (e.g. tracrRNA or an active partial tracrRNA), a tracr mate sequence (encompassing a "direct repeat" and a processed partial direct repeat in the context of an endogenous CRISPR system), a guide sequence or other sequences and transcripts from a CRISPR locus. In general, a CRISPR system is characterized by elements that promote the formation of a CRISPR complex at the site of a target sequence (also referred to as a protospacer in the context of an endogenous CRISPR system).

The formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. The tracr sequence (which may comprise or consist of all or a portion of a wild-type tracr sequence (e.g. about or more than about 20, 26, 32, 45, 48, 54, 63, 67, 85, or more nucleotides of a wild-type tracr sequence)), may also form portion of a CRISPR complex, such as by hybridization along at least a portion of the tracr sequence to all or a portion of a tracr mate sequence that is operably linked to the guide sequence. In some embodiments, the tracr sequence has sufficient complementarity to a tracr mate sequence to hybridize and participate in formation of a CRISPR complex. In some embodiments, the tracr sequence has at least 50%, 60%, 70%, 80%, 90%, 95% or 99% of sequence complementarity along the length of the tracr mate sequence when optimally aligned. In some embodiments, one or more vectors driving expression of one or more elements of a CRISPR system are introduced into a host cell such that expression of the elements of the CRISPR system direct formation of a CRISPR complex at one or more target sites.

In general, a tracr mate sequence comprises sufficient complementarity with a tracr sequence to promote formation of a CRISPR complex at a target sequence, wherein the CRISPR complex comprises the tracr mate sequence that hybridizes to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for the effects of secondary structures, such as self-complementarity within either the tracr sequence or tracr mate sequence. The degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. The tracr sequence is about or more than about 5, 6, 7, 8, 9, 10, 11, 12, 13, 14, 15, 16, 17, 18, 19, 20, 25, 30, 40, 50, or more nucleotides in length.

Non-limiting examples of Cas proteins include: Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. In some embodiments, the Cas protein is a Cas9 protein.

Cas9, also known as Csn1, is a large protein involved in both crRNA biosynthesis and in destruction of invading DNA. Cas9 has been described in different bacterial species such as *S. thermophiles, Listeria innocua* (Gasiunas, Barrangou et al. 2012; Jinek, Chylinski et al. 2012) and *S. Pyogenes* (Deltcheva, Chylinski et al. 2011). The large Cas9 protein (> 1200 amino acids) contains two predicted nuclease domains, namely HNH (McrA-like) nuclease domain that is located in the middle of the protein and a split RuvC-like nuclease domain (RNAase H fold) (Makarova, Grishin et al. (2006)). Cas9 variant can be a Cas9 endonuclease that does not naturally exist in nature and that is obtained by protein engineering or by random mutagenesis. Cas9 variants of the present invention can for example be obtained by mutations i.e. deletions from, or insertions or substitutions of at least one residue in the amino acid sequence of an *S. Pyogenes* Cas9 endonuclease (COG3513). In some embodiments, the Cas9 protein is *S. pneumoniae, S. pyogenes,* or *S. thermophiles* Cas9, and may include mutated Cas9 derived from these organisms, or variants with other amino acid sequences linked to Cas9, such as those with FokI enzyme linked to Cas9. These Cas9 are known; for example, the amino acid sequence of *Streptococcus pyogenes* Cas9 protein may be found in the SwissProt database under the accession number Q99ZW2; the amino acid sequence of *Neisseria meningitides* Cas9 protein may be found in the UniProt database under number A1IQ68; the amino acid sequence of *Streptococcus thermophilus* Cas9 protein may be found in the UniProt database under number Q03LF7; and the amino acid sequence of *Staphylococcus aureus* Cas9 protein may be found in the UniProt database under number J7RUA5.

### Guide RNA

In one aspect, the RNA component contained in the CRISPR is sometimes referred to as a guide RNA (gRNA). A guide RNA generally comprises a guide sequence and a backbone sequence, wherein the guide sequence and the backbone sequence may be in the same molecule or in different molecules. The role of the guide RNA is to direct the Cas9 protein to cleave the DNA site complementary to the guide sequence, i.e., the target sequence. In general, a guide sequence is any polynucleotide sequence having sufficient complementarity with a target polynucleotide sequence to hybridize with this target sequence and direct sequence-specific binding of a CRISPR complex to the target sequence. The degree of complementarity between a guide sequence and its corresponding target sequence is about or more than about 50% or more. In general, a guide sequence is about or more than about 12 nucleotides in length. The backbone sequence is necessary for a guide RNA. The other sequences other than the guide sequence generally comprise a tracr sequence and a tracr mate sequence, which generally do not vary with changes in the target sequence.

The guide RNA of the present invention includes single-stranded guide RNA (sgRNA) and double-stranded guide RNA consisting of crRNA and tracrRNA. In certain embodiments, the guide RNA has a double-stranded structure consisting of a crRNA (CRISPR RNA) and a tracrRNA (trans-activated crRNA). CrRNA generally comprises a guide sequence and a tracr mate sequence, and a tracrRNA generally comprises a tracr sequence. In some embodiments, the guide RNA is a single-stranded molecule that generally comprises a guide sequence, a tracr mate sequence, and a tracr sequence, and the single-stranded molecule is also referred to as a chimeric single-stranded guide RNA (sgRNA). When the tracr sequence and tracr mate sequence are contained within a single transcript, the hybridization between the two produces a transcript having a secondary structure (such as a hairpin). Preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In one embodiment, the transcript or transcribed polynucleotide sequence has at least two or more hairpins. In preferred embodiments, the transcript has two, three, four or five hairpins. In a further embodiment, the transcript has at most five hairpins. In some embodiments, the single transcript further includes a transcription termination sequence; preferably this is a Poly-U sequence, for example six U nucleotides.

In some embodiments, one or more vectors driving expression of one or more elements of a CRISPR system are introduced into a host cell, and the guide RNA enables expression of the elements of the CRISPR system to direct formation of a CRISPR complex at one or more target sites.

### SgRNA

An sgRNA is a chimeric single-stranded guide RNA that generally comprises a guide sequence, a tracr mate sequence, and a tracr sequence.

The first single-stranded guide RNA sequence of the present invention is 5'-(X)n-GUCUCUCAGCUGGUACA-backbone sequence-3', wherein X is any base selected from A, U, C and G, and n is any integer from 0 to 15, preferably n = 13, more preferably n = 2; and the backbone sequence generally comprises a tracr mate sequence and a tracr sequence. The structure of the backbone sequence is generally as described in figures 1A and 1B, figures 3A, 3B and 3C, and figures 4A, 4B, 4C, 4D and 4E of the document (Nowak et al. Nucleic Acids Research 2016. 44: 9555-9564), except for the content about the spacer sequence.

The potential backbone sequence is known to be any of SEQ ID NOs: 26-35 (listed as 5' to 3'), wherein the first block of lower case letters represent the tracr mate sequence, and the second block of lower case letters represent the tracr sequence, and the final poly-U sequence represents the transcription terminator. The number of Us in the poly-U is not limited to that shown in the example, and may be increased or decreased. In some embodiments, the poly-U may be removed without affecting the activity. In some embodiments, backbone sequences having about or more than about 50%, 60%, 70%, 80%, 90% or more similarity with the following sequences can be used. In some embodiments, the tracr sequence is a separate transcript from a transcript comprising the tracr mate sequence. In some embodiments, a chimeric single-stranded guide RNA (sgRNA) can be designed to incorporate a duplex structure of at least 12 bp between the direct repeat and the tracrRNA. In some embodiments, a design comprising a key RNA secondary structure which binds to the Cas9 protein, but having a mutated sequence or containing an inserted sequence, can also be used, as described in the published documents (Nowak et al. Nucleic Acids Research 2016. 44:9555-9564 and Adamson et al. Cell 2016. 167: 1867-1882).
(1) guuuuagagcuaGAAAuagcaaguuaaaauaaggcuaguccgUUUUUUUU (SEQ ID NO: 27)
(2) guuuuagagcuaGAAAuagcaaguuaaaauaaggcuaguccgUUUUUUUU (SEQ ID NO: 27)
(3) guuuuagagcuaGAAAuagcaaguuaaaauaaggcuaguccguuaucaUUUUUUUU(SEQ ID NO: 28)
(4) guuuuagagcuaGAAAuagcaaguuaaaauaaggcuaguccguuaucaacuugaaaaagugUUUUUUUU (SEQ ID NO: 29)
(5) 
(5)	guuuuagagcuaugcugGAAAcagcauagcaaguuaaaauaaggcuaguccguuaucaacuugaaaaaguggca
ccgagucggugcUUUUUU
(6) 
(6)	guuucagagcuaugcugGAAAcagcauagcaaguugaaauaaggcuaguccguuaucaacuugaaaaaguggca

ccgagucggugcUUUUUU
(7) 
(7)	guuuuagagcuauGAAAauagcaaguuaaaauaaggcuaguccguuaucaacuugaaaaaguggcaccgagucg
gugcUUUUUU	(SEQ ID NO: 32)
(8) 
(8) guuuuagagcuaugcGAAAgcauagcaaguuaaaauaaggcuaguccguuaucaacuugaaaaaguggcaccga
gucggugcUUUUUU (SEQ ID NO: 33)
(9) 
(9) guuuuagagcuaugcuguuuGAAAaaacagcauagcaaguuaaaauaaggcuaguccguuaucaacuugaaaaa
guggcaccgagucggugcUUUUUU (SEQ ID NO: 34)
(10)

(10) guuuuagagcuaugcuguuuugGAAAcaaaacagcauagcaaguuaaaauaaggcuaguccguuaucaacuuga
aaaaguggcaccgagucggugcUUUUUU (SEQ ID NO:35)

### crRNA and tracrRNA

In certain embodiments, the guide RNA has a double-stranded structure consisting of a crRNA and a tracrRNA. CrRNA generally comprises a guide sequence and a tracr mate sequence, and a tracrRNA generally comprises a tracr sequence. In general, the guide sequence has some complementarity with the target sequence, and the tracr mate sequence has some complementarity with the tracr sequence. The structure of the tracr mate sequence (also referred to as a repeat sequence) and the tracr sequence is generally as described in figure 1A of the document (Nowak et al. Nucleic Acids Research 2016. 44: 9555-9564) except for the content about the spacer sequence; and the complementary portion of the tracr mate sequence and the tracr sequence can be truncated by 1 to 10 base pairs and still have good activity, as described in figure 2B of the document (Cong et al. Science 2013. 339: 819-23), except for the content about the spacer sequence and the guide sequence.

The crRNA in the double-stranded guide RNA sequence that binds to the target sequence of SEQ ID NO: 2 comprises 5' (X)n-cacgcuggauagccucc-truncated backbone sequence-3'; the crRNA in the double-stranded guide RNA sequence that binds to the target sequence of SEQ ID NO: 3 comprises 5' (X)n-uagcgcgagcacagcua-truncated backbone sequence-3'; and the crRNA in the double-stranded guide RNA sequence that binds to the target sequence of SEQ ID NO: 4 comprises 5'-(X)n-cacggagcgagacaucu-truncated backbone sequence-3'; in which X is any base selected from A, U, C and G, and n is any integer from 0 to 15, preferably n = 13, more preferably n = 2.

An example of a potential truncated backbone sequence can be any of SEQ ID Nos: 36-45 (listed as 5' to 3'). In some embodiments, sequences having about or more than about 50%, 60%, 70%, 80%, 90% or more similarity with the following sequences can be used. In some embodiments, the 3' end (downstream) of these sequences may be added with at least 12 bases.

An example of a known tracrRNA sequence may be any one of SEQ ID Nos: 46-65 (listed as 5' to 3'), and the last poly-U sequence represents a transcription terminator. The number of Us in the poly-U is not limited to that shown in the example, and may be increased or decreased. In some embodiments, the poly-U may be removed without affecting the activity. In some embodiments, sequences having about or more than about 50%, 60%, 70%, 80%, 90% or more similarity with the following sequences can be used. In some embodiments, the 5' end (upstream) of these sequences may be added with at least 12 bases that are inversely complementary by a certain degree to the sequence at the 3' end of the truncated backbone. In some embodiments, a design comprising a key RNA secondary structure which binds to the Cas9 protein, but having a mutated sequence or containing an inserted sequence, can also be used, as described in the published documents (Nowak et al. Nucleic Acids Research 2016. 44:9555-9564; Adamson et al. Cell 2016. 167: 1867-1882).

### Target sequence

In the context of the formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridization and promote formation of a CRISPR complex. The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, the target sequence should be associated with a PAM (protospacer adjacent motif); that is, the target sequence should be associated with a short sequence recognized by the CRISPR complex.

The target sequence targeted by the CRISPR/Cas system of the present invention is a target sequence having SEQ ID NO: 1 in the TRAC gene, and a target sequence having SEQ ID NO: 2, SEQ ID NO: 3 and SEQ ID NO: 4 in the B2M gene. These target sequences are unique in the human genome.

### B2M gene

Beta-2 microglobulin (i.e., B2M) is the light chain of MHC class I molecules and is therefore an integral portion of the major histocompatibility complex (MHC). In the human genome, B2M is encoded by the b2m gene located on chromosome 15, while other MHC genes are present as gene clusters on chromosome 6. The human B2M protein has 119 amino acids (see UniProt database number P61769). In a model of mice lacking β-2 microglobulin, it can be demonstrated that B2M is essential for the presentation on the cell surface and the stability of the polypeptide binding groove of MHC class I molecules. MHC class I molecules are present on the surface of all nucleated cells in the human body. Mismatches in MHC can cause immune rejection, resulting in graft destruction. Removal of MHC class I molecules on the cell surface by knocking out B2M genes can prevent mismatches.

### TRAC gene

T cell receptor (TCR) is a receptor on the surface of T cells involved in the activation process of T cells after the T cells come into contact with the presented antigen. In general, TCR consists of two chains, an alpha chain and a beta chain, which form a heterodimer, and forms a T cell receptor complex on the cell surface together with the CD3 molecule. Each alpha or beta chain contains a variable region and a constant region, wherein the constant region of the alpha chain is encoded by a TRAC gene located on chromosome 14. TCR can recognize a processed polypeptide fragment that binds to an MHC molecule, which is also called MHC restriction because the recognition requires the presentation of MHC molecules. When the MHC molecules of the donor and recipient are different, the TCR can recognize the difference in MHC and cause activation and expansion of T cells, possibly resulting in graft versus host disease (GvHD). Knocking out TRAC genes can remove the expression of the TCR alpha chain, thereby removing the TCR from the surface of T cells and thus preventing graft versus host disease caused by TCR recognition of allogeneic antigens.

### Delivery method

The molecules used in the present invention can be delivered intracellularly by any method known in the art. Non-limiting examples include viral transduction, transfection by electroporation, liposome delivery, polymeric carriers, chemical carriers, lipid complexes, polymeric complexes, dendrimers, nanoparticles, emulsions, a natural endocytosis or phagocytosis pathway, a cell penetrating peptide, microinjection, microneedle delivery, particle bombardment, etc.

A preferred embodiment is transfection by electroporation, and non-limiting examples of instruments which can be used in transfection by electroporation include: Neon transfection system (Thermo Fisher Scientific), Gemini instrument and AgilePulse/CytoPulse instrument (BTX-Harvard apparatus), 4D-Nucleofector system, Amaxa Nucleofector II, Nucleofector 2b instrument (Lonza), CTX-1500A instrument (Celetrix), MaxCyte GT or VLX instrument (MaxCyte), and Gene Pulser Xcell (Biorad). Based on the guidance of manufacturers, the duration and intensity of pulses, interval between pulses, number of pulses, and optimal conditions for high transfection efficiency with low mortality can be modified.

As a preferred embodiment, in the electroporation, 0.05 to 1000 µg of Cas9 protein or mRNA, or 0.01 to 2000 µg of gRNA; preferably, 0.2-20 µg of Cas9 protein or mRNA, 0.04-40 µg of gRNA can be used per 10E6 of cells. The transfection is carried out at 4°C to 37°C, preferably at 4°C or room temperature (20°C-25°C); the buffer used may be Neon system T buffer (Thermo, MPK1096), BTXpress electroporation buffer (BTX, 45-0802), Opti-MEM medium (Thermo, 51985042), Cytoporation medium T (BTX, 47-0002) and T4 (BTX, 47-0003), and any other buffers with similar conductivity to the above-mentioned buffers.

In the embodiments of the present invention, the main types of transfected cells comprise human primary T cells, lymphocytes, and peripheral blood mononuclear cells. In theory, vectors can be delivered into most types of cells using the method of transfection by electroporation. Preferably, lymphocytes from a single donor or a mixture of lymphocytes from multiple donors can be transfected. Since primary lymphocytes are taken from the peripheral blood of human donors in most cases, the maximum number of cells that can be obtained from a single donor at a time is limited, which causes a limit to the total number of cells that can be processed per batch. If a mixture of cells from multiple donors can be transfected, so that cells processed in a single batch are not necessarily from the same donor, the total number of cells that can be processed can be multiplied, which increases the number of cells that can be produced in a single batch and expands production scale, thereby reducing the cost of production.

### Preparation of chimeric antigen receptor (CAR) T cells

The CRISPR-Cas system of the present invention can be used to prepare T cells expressing a chimeric antigen receptor (CAR). Inactivation of the B2M and TRAC genes in T cells by the gene editing of the present invention can effectively reduce the immune rejection of CAR-T cells in the treatment of allogeneic cells.

The T cells to be modified according to the present invention may be any suitable T cells. For example, the T cells can be inflammatory T lymphocytes, cytotoxic T lymphocytes, regulatory T cells, or helper T lymphocytes. Specifically, the T cells are cytotoxic T lymphocytes. In certain embodiments, the T cells are selected from CD4+ T lymphocytes and CD8+ T lymphocytes. They can be extracted from the blood or derived from stem cells. The stem cells may be adult stem cells, embryonic stem cells, more particularly non-human stem cells, umbilical cord blood stem cells, progenitor cells, bone marrow stem cells, induced pluripotent stem cells, totipotent stem cells or hematopoietic stem cells. Representative human cells are CD34+ cells. In a specific embodiment, the T cells to be modified according to the present invention are human T cells. Prior to expansion and genetic modification of the cells of the present invention, a source of cells can be obtained from a subject (e.g. a patient) through a variety of non-limiting methods. T cells can be obtained from a number of non-limiting sources, including peripheral blood mononuclear cells, bone marrow, lymph node tissue, umbilical cord blood, thymus tissue, tissue from a site of infection, ascites, pleural effusion, spleen tissue, and tumors. In certain embodiments of the present invention, any number of T cell lines, available and known to a person skilled in the art, may be used. In another embodiment, the cell can be derived from a healthy donor, from a patient diagnosed with cancer or from a patient diagnosed with an infection. In another embodiment, the cell is a portion of a mixed population of cells which present different phenotypic characteristics.

### Examples

The examples are merely illustrative and are not intended to limit the present invention in any way.

### Example 1: Design of target sequence and guide RNA

A human T cell receptor (TCR) αβ is a dimer composed of an α chain and a β chain. TRAC gene is a constant (C) region of the alpha chain which binds to other parts of the alpha chain, including variable regions, during RNA splicing to form a full length alpha chain. Since the sequence of the constant region TRAC is invariant in the TCR, we designed a CRISPR/Cas9 guide RNA for the coding exon of the TRAC gene. We searched for the coding exons 1, 2 and 3 of the human TRAC gene, which are 273 bp, 45 bp and 108 bp, respectively (Genbank number: M94081.1, 87701..92337/gene="Tcr-C-alpha"), identified all possible target sequences, and excluded some target sequences that may produce more severe off-target effects in the genome. The target sequence was searched for as follows: identifying all the sequences in the form of 5'-NNNNNNNNNNNNNNNNNNN-NGG-3' (N is A, C, G, or T) on the sense and antisense DNA strands, where "NGG" is PAM and NNNNNNNNNNNNNNNNNNN before NGG is the target sequence, also known as the protospacer. After testing, it was found that the guide RNA (gRNA) binding to the target sequence numbered 1-8 has the highest TCR gene knockout activity in primary T cells (Table 1).

The human B2M gene encodes a beta-2 microglobulin, which protein is a non-polymorphic portion of an MHC class I molecule (also known as a human leukocyte antigen), similar to the stable region of an immunoglobulin, and thus suitable for use as a target for gene editing. Similar to the above-described design process of gRNA of the TRAC gene, we searched for the coding exons 1 and 2 of the B2M gene, which are 67 bp and 279 bp, respectively (Genbank number: NC_000015.10, REGION: 44711487..44718159), identified all possible target sequences, and excluded some target sequences that may produce more severe off-target effects in the genome. The target sequence was searched for as follows: identifying all the sequences in the form of 5'-NNNNNNNNNNNNNNNNNNN-NGG-3' (N is A, C, G, or T) on the sense and antisense DNA strands, where "NGG" is PAM and NNNNNNNNNNNNNNNNNNN before NGG is the target sequence. After testing, 2-2, 2-4, 2-6 and 2-27 were screened for gRNA with a relatively high activity (Table 1).

**Table 1: Target sequences of CRISPR/Cas9 in the TRAC and B2M genes. The 3' end of each target sequence in the genome is followed by "5'-NGG"PAM.**

| No. | Target sequence (5'-3') | SEQ ID NO. | Sense (+) or antisense (-) chain | Target gene | Exon |
|---|---|---|---|---|---|
| 1-8 | GAGTCTCTCAGCTGGTACA | 1 | - | TRAC | 1 |
| 2-2 | CTCACGCTGGATAGCCTCC | 2 | - | B2M | 1 |
| 2-4 | AGTAGCGCGAGCACAGCTA | 3 | - | B2M | 1 |
| 2-6 | GCCACGGAGCGAGACATCT | 4 | - | B2M | 1 |
| 2-27 | AAGTTGACTTACTGAAGAA | 5 | + | B2M | 2 |

In this example, DNA target sequence before "NGG" (PAM) on the genome corresponding to the guide sequence of the gRNA is 19 bases, and the form with "NGG" (PAM) in the genome is 5'-NNNNNNNNNNNNNNNNNNN-NGG-3'. It is shown in documents that gRNAs corresponding to the target sequence of 17 to 30 bases before "NGG" have efficient cleavage efficiency, and approximately 10 bases at the 5' end of the guide sequence of 30 bases are removed in the cell. Therefore, the sequences of said about 10 bases in the gRNA of the guide sequence of 30 bases has no effect on the cleavage efficiency, that is, the guide sequence only needs to correspond to the 17 bases immediately before the 5' end closest to the PAM, and 0 to 13 bases of any sequence at the 5' end can be added without affecting the cleavage efficiency (Ran et al. Cell 2013. 154: 1380-9. Figure 1; Fu et al. Nat Biotechnol 2014. 32: 279-84). Therefore, the guide sequence of 19 bases shown in the present invention can be cleaved to the guide sequence of 18 or 17 bases (removing 1 or 2 bases at the 5' end) or be the guide sequence in the form of 5'-(X)n-NNNNNNNNNNNNNNNNN by removing 2 bases at the 5' end and adding 0 to 13 bases of any sequence (X is an arbitrary base; (X)n means that there are n Xs, where n can be any numerical value of 0, 1, 2, 3, 4, 5, 6, 7, 8, 9, 10, 11, 12, 13 or represent more bases; NNNNNNNNNNNNNNNNN is an RNA sequence corresponding to the target sequence before the 5 'end of the PAM in the genome), wherein these changes are allowed in the implementation. For example, the target sequence numbered 1-8 is 5'- GAGTCTCTCAGCTGGTACA-3'(SEQ ID NO:1); the guide sequence of the corresponding gRNA is 5'-GAGUCUCUCAGCUGGUACA-3'; the sequence of 18 bases cleaved from the target sequence is 5'-AGUCUCUCAGCUGGUACA-3'; and the sequence of 17 bases cleaved from the target sequence is 5'-GUCUCUCAGCUGGUACA-3'; the guide sequence can be in the form of 5'-(X)n-GUCUCUCAGCUGGUACA-3' by adding n arbitrary bases X, where n can be any numerical value of 0, 1, 2, 3, 4, 5, 6, 7, 8, 9, 10, 11, 12, 13 or represent more bases.

### Example 2: Preparation of CRISPR/Cas9 RNP system

We purchased Cas9 proteins (SpCas9) derived from wild-type *Streptococcus pyogenes* and having a nuclear localization signal (NLS) from different manufacturers, including Integrated DNA Technologies (Art. No. 1074181) and PNA Bio (Art. No. CP02-250). The amino acid sequence of Cas9 protein with N-terminal and C-terminal nuclear localization signals is as shown in SEQ ID NO: 6.

The CRISPR/Cas9 system in the examples is derived from *S. pyogenes.* The chimeric single-stranded guide RNA (sgRNA) commonly used in this system consists of two portions, a guide sequence in which the first portion of the 5 'end contains 19 bases corresponding to the target sequence (the guide sequence has the same 19 bases as those immediately before the 5' end of the PAM, except the guide sequence is an RNA strand, that is, T is replaced by U), and a backbone sequence binding to the second portion, which backbone sequence is 5'-GUUUUAGAGCUAGAAAUAGCAAGUUAAAAUAAGGCUAGUCCGUUAUC AACUUGAAAAAGUGGCACCGAGUCGGUGCUUUU-3' (SEQ ID NO: 7). The number of base U at the end of the backbone sequence can vary. For example, the target sequence numbered 1-8 (table 1) is 5'- GAGTCTCTCAGCTGGTACA-3'(SEQ ID NO:1); the guide sequence of the corresponding sgRNA is 5'-GAGUCUCUCAGCUGGUACA-3'; and the full sequence of the sgRNA is 5'-GAGUCUCUCAGCUGGUACAGUUUUAGAGCUAGAAAUAGCAAGUUAAA AUAAGGCUAGUCCGUUAUCAACUUGAAAAAGUGGCACCGAGUCGGUG CUUUU -3'(SEQ ID NO: 8).

We used a modified version of the backbone sequence with an extended complementary region in the sgRNA used in the examples (Dang et al. Genome Biology 2015. 16: 280), wherein the backbone sequence is: 5'-GUUUUAGAGCUAUGCUGGAAACAGCAUAGCAAGUUAAAAUAAGGCUA GUCCGUUAUCAACUUGAAAAAGUGGCACCGAGUCGGUGCUUUU-3' (SEQ ID NO: 9). We synthesized the sgRNA required for the experiment by in vitro transcription (IVT). Firstly, a DNA template with a T7 promoter (see the underlined portion of the primer sequences) required for in vitro transcription was generated by a PCR reaction. The sequences of 2 PCR primers overlapping each other are as follows: the upstream primer is 5'- TAATACGACTCACTATAGG-(target sequence, as shown in Table 1)-GTTTTAGAGCTATGCTG-3' (SEQ ID NO: 10), and the downstream primer is 5'-AAAAGCACCGACTCGGTGCCACTTTTTCAAGTTGATAACGGACTAGCCTT ATTTTAACTTGCTATGCTGTTTCCAGCATAGCTCTAAAAC-3' (SEQ ID NO: 11). 2 G bases were added to the end of the above-mentioned T7 promoter to ensure efficient transcription. When the sgRNA corresponding to each different DNA target sequence is produced, the sequence within the bracket in the middle of the upstream primer thereof is the target sequence (without PAM), while the sequence of the downstream primer is shared and does not vary due to different target sequences. For example, the target sequence numbered 1-8 in table 1 is 5'-GAGTCTCTCAGCTGGTACA-3' (SEQ ID NO:1); the sequence of the upstream primer used to produce the transcription template for the sgRNA is 5'-TAATACGACTCACTATAGG-GAGTCTCTCAGCTGGTACA-GTTTTAGAGCTATGCTG-3' (SEQ ID NO: 12) and the sequence of the downstream primer is, for example, SEQ ID NO: 11. The sequence of the PCR product using these 2 primers, that is, the sequence numbered 1-8 of the in vitro transcription template of the sgRNA is 5'- TAATACGACTCACTATAGG-GAGTCTCTCAGCTGGTACA-GTTTTAGAGCTATGCTGGAAACAGCATAGCAAGTTAAAATAAGGCTAGTC CGTTATCAACTTGAAAAAGTGGCACCGAGTCGGTGCTTTT-3' (SEQ ID NO: 13).

The PCR reaction was carried out using the above-mentioned 2 primers, upstream primer and downstream primer, and Q5 Hot Start High-Fidelity 2X Master Mix kit (New England Biolabs, M0494S), wherein the final concentration of each primer is 0.5 µM. 10 µl of Q5 2X Master Mix was added and then water was added to a total volume of 20 µl; and PCR was carried out according to the instructions of manufacturers.

The next in vitro transcription reaction was carried out using 8 µl of the above-mentioned PCR product as a DNA template, and using HiScribe T7 Quick High Yield RNA Synthesis Kit (New England Biolabs, E2050S) according to instructions of manufacturers in a total of 20 µl of the reaction system. After incubating for 2 hours at 37°C, 1 µl of DNase was added and incubation was continued at 37°C for 15 minutes to remove the DNA template. The sgRNA produced by the reaction was then purified using Agencourt RNAClean XP magnetic beads (Beckman-Coulter, A63987) and eluted with nuclease-free water or duplex buffer (Integrated DNA Technologies (IDT), Art. No. 11-01-03-01) and stored at -20°C or -80°C.

**Table 2: sgRNA sequences corresponding to different target sequences, and upstream and downstream primers used to prepare the sgRNA in vitro transcription template.**

| No. | sgRNA sequence (lower case letters represent guide sequences) | Upstream primer | Downstream primer |
|---|---|---|---|
| 1-8 | gagucucucagcugguaca GUUUUAGAGCUAUGCU GGAAACAGCAUAGCAA GUUAAAAUAAGGCUAG UCCGUUAUCAACUUGA AAAAGUGGCACCGAGU CGGUGCUUUU (SEQ ID NO: 14) | TAATACGACTCACTATAGG GAGTCTCTCAGCTGGTAC A GTTTTAGAGCTATGCTG (SEQ ID NO: 12) | AAAAGCACCGAC TCGGTGCCACTTT TTCAAGTTGATAA CGGACTAGCCTTA TTTTAACTTGCTA TGCTGTTTCCAGC ATAGCTCTAAAAC (SEQ ID NO: 11) |
| 2-2 | cucacgcuggauagccucc GUUUUAGAGCUAUGCU GGAAACAGCAUAGCAA GUUAAAAUAAGGCUAG UCCGUUAUCAACUUGA AAAAGUGGCACCGAGU CGGUGCUUUU (SEQ ID NO: 15) | TAATACGACTCACTATAGG CTCACGCTGGATAGCCTC C GTTTTAGAGCTATGCTG (SEQ ID NO: 16) | AAAAGCACCGAC TCGGTGCCACTTT TTCAAGTTGATAA CGGACTAGCCTTA TTTTAACTTGCTA TGCTGTTTCCAGC ATAGCTCTAAAAC (SEQ ID NO: 11) |
| 2-4 | aguagcgcgagcacagcua GUUUUAGAGCUAUGCU GGAAACAGCAUAGCAA GUUAAAAUAAGGCUAG UCCGUUAUCAACUUGA AAAAGUGGCACCGAGU CGGUGCUUUU (SEQ ID NO: 17) | TAATACGACTCACTATAGG AGTAGCGCGAGCACAGCT A GTTTTAGAGCTATGCTG (SEQ ID NO: 18) | AAAAGCACCGAC TCGGTGCCACTTT TTCAAGTTGATAA CGGACTAGCCTTA TTTTAACTTGCTA TGCTGTTTCCAGC ATAGCTCTAAAAC (SEQ ID NO: 11) |
| 2-6 | gccacggagcgagacaucu GUUUUAGAGCUAUGCU GGAAACAGCAUAGCAA GUUAAAAUAAGGCUAG UCCGUUAUCAACUUGA AAAAGUGGCACCGAGU CGGUGCUUUU (SEQ ID NO: 19) | TAATACGACTCACTATAGG GCCACGGAGCGAGACATC T GTTTTAGAGCTATGCTG (SEQ ID NO: 20) | AAAAGCACCGAC TCGGTGCCACTTT TTCAAGTTGATAA CGGACTAGCCTTA TTTTAACTTGCTA TGCTGTTTCCAGC ATAGCTCTAAAAC (SEQ ID NO: 11) |
| 2-27 | aaguugacuuacugaagaa GUUUUAGAGCUAUGCU GGAAACAGCAUAGCAA GUUAAAAUAAGGCUAG UCCGUUAUCAACUUGA AAAAGUGGCACCGAGU CGGUGCUUUU (SEQ ID NO: 21) | TAATACGACTCACTATAGG AAGTTGACTTACTGAAGA A GTTTTAGAGCTATGCTG (SEQ ID NO: 22) | AAAAGCACCGAC TCGGTGCCACTTT TTCAAGTTGATAA CGGACTAGCCTTA TTTTAACTTGCTA TGCTGTTTCCAGC ATAGCTCTAAAAC (SEQ ID NO: 11) |

In addition to the form of single-stranded sgRNA, we also used a system of double-stranded RNA, that is, a complex of crRNA and tracrRNA binds to the Cas9 protein. Commonly-used double-stranded RNA sequences are as follows: crRNA is 5'- (guide sequence of 19 bases, see the part of lower case letters in table 2)-GUUUUAGAGCUAUGCU-3' (SEQ ID NO: 23); tracrRNA is 5'-AGCAUAGCAAGUUAAAAUAAGGCUAGUCCGUUAUCAACUUGAAAAAG UGGCACCGAGUCGGUGCUUU-3'(SEQ ID NO: 24). For example, the target sequence numbered 1-8 is 5'- GAGTCTCTCAGCTGGTACA-3' (SEQ ID NO: 1); the sequence numbered 1-8 of the crRNA is 5' GAGUCUCUCAGCUGGUACA-GUUUUAGAGCUAUGCU-3 '(SEQ ID NO: 25).

These single-stranded sgRNA or double-stranded crRNA and tracrRNA sequences can have certain bases thereof extended, cleaved, or slightly altered based on the crRNA and tracrRNA forms of *S. pyogenes* in nature, while still maintaining similar activities, as described in, for example, patent US 8,697,359 B1 and patent application WO 2016/100951.

Prior to use with the Cas9 protein, crRNA and tracrRNA require formation of a double-stranded complex. For example, the two RNAs can be mixed in the same molar ratio in nuclease-free duplex buffer (IDT, Art. No. 11-01-03-01), heated to 95°C for 5 minutes, and then cooled at room temperature (20°C-25°C).

### Example 3: Efficiency of Cas9/sgRNA in knockout of TCR and MHC class I in primary T cells

Peripheral blood mononuclear cells (PBMC) are isolated and purified from whole blood of healthy human donors using conventional Ficoll density gradient centrifugation according to the instructions of manufacturers. Lymphoprep separation liquid (StemCell Technologies, 07851) is used in this example. The isolated and purified PBMCs are cultured in T cell medium (Takara, GT-T551) supplemented with IL-2 (PeproTech, 200-02), and activated using CD3/CD28 antibody magnetic beads Dynabeads (Thermo Fisher Scientific, 1141D). After co-incubation with magnetic beads for 3 days in a 37°C cell culture incubator, approximately 80% of PBMCs became CD3-positive T cells (the detection method is described below).

The transfection by electroporation (electrotransfection) is conducted on day 3 after starting incubation of magnetic beads. The CRISPR/Cas9 RNP complex needs to be prepared before using the Neon transfection system (Thermo Fisher Scientific) for electrotransfection of the T cells which are activated with magnetic beads for 3 days beforehand. A total of 0.4 µg of a single sgRNA transcribed in vitro, or a combination of 2-3 sgRNAs which are mixed in an equal mass ratio and have a constant total mass (see example 2 for sgRNA preparation, and tables 3 and 4 for gRNA combinations) and 1 µg of Cas9 protein (purchased from IDT) are mixed in T buffer provided in a Neon kit (Thermo, MPK1096), with a total volume of 6 µl; the mixture is incubated at room temperature for 5 to 10 minutes, and then mixed with 6 µl of T cells (0.2x10E6) resuspended in the T buffer, and then subjected to electrotransfection in a 10 µl pipette tip of the Neon according to the instructions for T cell electrotransfection provided by manufacturers. The method for electrotransfection is described in the document (Schumann et al. PNAS 2015. 112: 10437-10442).

4 days after the electrotransfection, cells are stained with APC fluorescently-labeled CD3 antibody (BD Bioscience, 555335) and PE fluorescently-labeled B2M antibody (BD Bioscience, 551337) to measure the deletion of TCR and MHC class I molecules in the cell surface caused by gene editing. This method is used to measure the percentage of each gene knocked out by the CRISPR/Cas9, and the knockout percentages described below are all measured using the antibody staining method described herein. Since CD3 molecules form a complex with TCR molecules, CD3 is not presented to the cell surface without TCR, and CD3 antibodies can be used to measure whether TCR is knocked out by the CRISPR/Cas9 that targets TRAC genes (the constant region of the alpha chain of TCR). B2M is a component of MHC class I molecules, so B2M staining can indicate whether MHC class I molecules are knocked out. Since the staining of control cells of which control RNAs have been subjected to electrotransfection (Trilink, L-6101) shows that most cells have CD3 and B2M on the surface when the CRISPR/Cas9 is not added (Figure 1), the loss of CD3 or B2M in samples having electrotransfected Cas9 and gRNA is caused by the gene knockout of the CRISPR/Cas9. On the day of the electrotransfection, 80% of the cells are CD3-positive T cells; CD3-positive T cells reach nearly 90% of the cells at 5 days after the electrotransfection of the negative control RNA (Trilink, L-6101) (Figure 1). Therefore, most of the cells that are processed during and after the electrotransfection are primary T cells.

The tests were conducted in the T cells by the above-mentioned method, it was found that the sgRNA binding to the target sequence numbered 1-8 has the highest TCR gene knockout activity in T cells, and an average of 83% ± 3% of CD3 negative cells (i,e., cells that are completely knocked out by TCR) were obtained in three independent experiments. The sgRNAs with a relatively high activity against B2M genes are numbered 2-2, 2-4, 2-6 and 2-27, and respectively achieved a ratio of B2M negative cells of 47% ± 13%, 54% ± 8%, 30% ± 3% and 66% ± 3% in T cells (data are mean ± standard deviations in 3 independent experiments).

In order to further improve the knockout efficiency of B2M, two gRNAs were used to cleave adjacent sites on the gene, such that a small portion of the sequence between the two adjacent sites may be lost after simultaneous cleavage, resulting in a greater probability of gene disruption. Table 3 shows that insofar as the total gRNA mass is the same, the efficiency of a combination of two gRNAs (each accounting for 1/2 of the total gRNA mass) is sometimes higher than that of either gRNA in the combination. In these combinations, 2-2/2-4, 2-2/2-6, and 2-4/2-6 combinations achieve a relatively high efficiency, and it is expected that these gRNA sequences have a relatively low off-target effect in the genome.

**Table 3: Efficiency of using gRNA combination of CRISPR/Cas9 in primary human T cells to knock out MHC class I molecules. B2M genes are knocked out using a composition of two gRNAs, resulting in the loss of class I MHC. The "B2M knockout percentage" is the ratio of B2M negative cells after electrotransfection of Cas9 and the gRNA combination shown in the table.**

| Groups | Targeted B2M exon | gRNA 1 | gRNA 2 | B2M knockout percentage (%) |
|---|---|---|---|---|
| Group 1 | 1 | 2-2 | 2-4 | 65 |
| Group 2 | 1 | 2-4 | 2-6 | 53 |
| Group 3 | 1 | 2-2 | 2-6 | 76 |
| Group 4 | 2 | 2-27 | 2-29 | 64 |
| Group 5 | 2 | 2-29 | 2-32 | 31 |
| Group 6 | 2 | 2-27 | 2-32 | 50 |

Different gRNAs that target TRAC and B2M can be combined to transfect T cells, thereby simultaneously knocking out the two genes in the cell, and removing TCR and MHC class I molecules on the cell surface. The method for electrotransfection is as described above. Where the total gRNA mass is constant at 0.4 µg, each of the composition of two gRNAs (if used) is 1/2 of the total mass of the gRNAs, that is, 0.2 µg, and each of the composition of three gRNAs (if used) is 1/3 of the total mass of the gRNAs, that is, 0.13 µg. (Figure 2). The results after transfection with different gRNA compositions are shown. The cells in the lower left quadrant of each panel are CD3-B2M-double negative cells, and the ratio of cells in this quadrant is the double-gene knockout percentage, wherein the higher the ratio, the better the effect of the gRNA combination. Table 4 summarizes the efficiency of these different gRNA combinations. It can be seen that 1-8/2-2/2-4 composition, 1-8/2-2/2-6 composition, and 1-8/2-4/2-6 composition all have a double knockout efficiency of > 40%.

**Table 4: Efficiency of using gRNA combination of CRISPR/Cas9 in primary human T cells to simultaneously knock out TCR and MHC class I molecules. The two genes, TRAC and B2M, are simultaneously knocked out when the composition of two or three gRNAs is used, and "double knockout percent" is the percentage of CD3-B2M-double negative cells after electrotransfection of Cas9 and the gRNA combinations shown in the table. Analyzing the panels of flow cytometry in this ratio (Figure 2).**

| Groups | Number of gRNAs used | gRNA 1 | gRNA 2 | gRNA 3 | Double knockout percentage (%) |
|---|---|---|---|---|---|
| Group 1 | 3 | 1-8 | 2-2 | 2-4 | 61.5 |
| Group 2 | 3 | 1-8 | 2-2 | 2-6 | 52.8 |
| Group 3 | 3 | 1-8 | 2-4 | 2-6 | 43.4 |
| Group 4 | 3 | 1-8 | 2-4 | 2-27 | 19.5 |
| Group 5 | 2 | 1-8 | 2-4 | | 22.4 |
| Group 6 | 2 | 1-8 | 2-27 | | 27.5 |

In order to test whether the efficient double-gene knockout has repeatability, the 1-8/2-2/2-4 compositions are tested in T cells from 4 different healthy human donors. The method described above is used, wherein the 3 sgRNAs are mixed with in a equal mass ratio, electrotransfected into the primary T cells together with Cas9 protein, and stained with fluorescent antibodies after 4 days of electrotransfection to obtain the double-gene knockout efficiency as shown in Table 5 below. The average double knockout percentage of the 4 donors reaches 55% ± 6% (mean ± standard deviation), which demonstrates the efficiency and reproducibility of removing GvHD and HvGR-associated molecules on the cell surface using this method.

**Table 5: Efficiency of using gRNAs numbered 1-8, 2-2 and 2-4 in a mass ratio of 1 : 1 : 1 in primary T cells from different healthy human donors to simultaneously knock out TCR and MHC class I molecules. "TCR knockout percentage" is the ratio of CD3 negative cells after electrotransfection; "B2M (MHC-I) knockout percentage" is the ratio of B2M negative cells; and "double knockout percentage" is the ratio of CD3-B2M-double negative cells.**

| Healthy human donor number | TCR knockout percentage (%) | B2M (MHC-I) knockout percentage (%) | Double knockout percentage (%) |
|---|---|---|---|
| 1 | 80.0 | 65.0 | 61.5 |
| 2 | 78.8 | 62.7 | 57.8 |
| 3 | 78.6 | 61.2 | 54.4 |
| 4 | 77.3 | 51.3 | 46.7 |

### Example 4: Efficient double-gene knockout in the mixture of activated primary T cells from different donors

In the production of allogeneic universal T cells, if the cells from different donors can be mixed and processed in batches, the cost of the production is greatly reduced. We therefore tested whether T cells from unrelated healthy donors could be mixed together and do not interfere with efficient downstream gene editing. As in example 3, Dynabeads magnetic beads (Thermo, 1141D) are used to respectively activate PBMCs from different healthy donors. After 3 days of activation, Dynabeads are removed using a magnetic stand, and the same number of activated cells from different donors are mixed together. The in vitro transcribed sgRNAs prepared by ourselves are combined (1-8/2-2/2-4) according to the method described in example 3 and transfected into mixed T cells from 2 or 3 donors with Cas9 protein purchased from IDT, wherein the total number of T cells remain unchanged, and the cells of each donor are mixed in equal proportions. 4 days after electrotransfection, the cells are stained with CD3 (BD Bioscience, 555335) and B2M (BD Bioscience, 551337) fluorescently-labeled antibodies and analyzed using a flow cytometer (Table 6). The comparison results show that the single gene/multiple gene-knockout efficiency of mixed cells from multiple donors is similar to that of cells from a single donor, indicating that the CRISPR/Cas9 system of the present invention can also produce universal cell preparations for mixed cells from multiple donors.

**Table 6: Efficiency of using gRNAs numbered 1-8, 2-2 and 2-4 in a mass ratio of 1 : 1 : 1 in mixed primary T cells from different healthy human donors to simultaneously knock out TCR and MHC class I molecules. "TCR knockout percentage" is the ratio of CD3 negative cells after electrotransfection; "B2M (MHC-I) knockout percentage" is the ratio of B2M negative cells; and "double knockout percentage" is the ratio of CD3-B2M-double negative cells.**

| Cell mix of healthy human donor (number) | TCR knockout percentage (%) | B2M (MHC-I) knockout percentage (%) | Double knockout percentage (%) |
|---|---|---|---|
| 1 and 2 mix | 66.1 | 55.7 | 47.1 |
| 1 and 3 mix | 62.0 | 45.5 | 38.6 |
| 2 and 3 mix | 72.4 | 51.3 | 45.4 |
| 1, 2 and 3 mix | 69.1 | 49.4 | 44.0 |

### Example 5: Activity of chemically modified synthetic guide RNA in primary T cells

It has been reported that chemical modification of RNA, such as cross-linking of 2'-O-methyl and 3'-phosphorothioate, can resistant degradation of RNA by intracellular environments such as nucleases (Kurreck. Eur J Biochem 2003. 270: 1628-1644; Behlke. Oligonucleotides 2008. 18:305-320; Lennox et al. Gene Therapy 2011. 18: 1111-1120). Therefore, chemically modified gRNAs are more stable in primary cells, thereby improving the efficiency of gene editing of the CRISPR/Cas9 system. Here we tested and compared the efficiency of gene editing of different forms of guide RNA-mediated CRISPR/Cas9 system in T cells by 4 methods. The 4 forms of guide RNAs are respectively: sgRNA obtained by in vitro transcription (see example 2 for preparation), chemically modified crRNA and tracrRNA purchased from IDT, synthetic sgRNA without chemical modification purchased from Synthego, and chemically modified synthetic sgRNA purchased from Synthego.

According to patent document WO 2016/100951, Alt-R CRISPR/Cas9 crRNA and tracrRNA from IDT contain a number of chemical modifications to prevent RNA degradation. We entered each target sequence of 19 bases in Table 1 into the order system of IDT to order the corresponding 35-base Alt-R crRNA oligonucleotide containing a guide sequence of 19 bases. According to the method described in example 2, before use, the 67 base Alt-R tracrRNA from IDT is used as a universal sequence to bind to each Alt-R crRNA to form an RNA double-stranded complex.

We obtained synthetic sgRNAs with or without chemical modification from Synthego. These sgRNAs have a backbone sequence of 80 bases. The structure of sgRNA from Synthego is as follows: 5'-(RNA guide sequence of 19 bases matching the target sequence in Table 1)-(backbone sequence of 80 bases from Synthego) -3'. The synthetic sgRNA without chemical modification has an identical RNA sequence to the chemically modified sgRNA. The chemically modified sgRNA has 2'-O-methyl and 3' phosphorothioate modifications at the last 3 bases of the 5' and 3' ends, respectively.

After binding the sgRNA or crRNA/tracrRNA duplex to the Cas9 protein, we transfected same into cells using the Neon system (Thermo) according to the method described in example 3. After 4 or 5 days of electrotransfection, we stained the cells with CD3 and B2M fluorescently-labeled antibodies and analyzed the stained cells using a flow cytometer. The results shown in Table 7 indicate that when the chemically modified guide RNA is used, the knockout efficiency, especially the double-gene knockout efficiency, is significantly improved.

**Table 7: Effects of 4 different forms of guide RNAs, with or without chemical modification, in human primary T cells. "Editing efficiency" is a value from the flow cytometry analysis, and refers to the ratio of CD3 negative cells for 1-8, the ratio of B2M negative cells for 2-4, and the ratio of CD3 and B2M double negative cells for 1-8/2-4/2-27 combination.**

| Guide RNA | Editing efficiency (%) of unmodified sgRNA obtained by in vitro transcription | Editing efficiency (%) of chemically modified crRNA+tracrRNA (from IDT) | Editing efficiency (%) of unmodified sgRNA (from Synthego) | Editing efficiency (%) of chemically modified sgRNA (from Synthego) |
|---|---|---|---|---|
| 1-8 | 71.3 | 80.2 | 79.9 | 78.0 |
| 2-4 | 40.8 | 56.2 | 43.3 | 58.4 |
| 1-8/2-4/2-27 (mixed in a mass ratio of 1 : 1 : 1) | 19.5 | 33.3 | 29.5 | 63.0 |

In order to further improve the efficiency of gene editing, we also increased the amount of Cas9 protein and guide RNA, and optimized the ratio of several guide RNAs in the guide RNA combination. We used the Neon system to transfect a total of 0.8 µg of crRNA/tracrRNA duplex (from IDT) and 2 µg of Cas9 protein (from IDT) into 200,000 activated primary T cells. Meanwhile, we tested a variety of crRNA/tracrRNA duplexes, including 1-8 alone, and 3 guide RNA combinations, 1-8/2-2/2-4. The transfection procedure is described in detail in example 3. In experiments using separate guide RNAs numbered 1-8, and under the conditions of no enrichment, the single-gene knockout efficiency for TCR is up to 90% or more (The cells in the lower right quadrant of Figure 3C are CD3-negative and B2M-positive cells, representing the cells with TCR single-gene knockout). In experiments using 3 guide RNA combinations, the mass ratios of three chemically modified guide RNAs (from IDT), 1-8, 2-2, and 2-4, became 2: 1: 1, and under the conditions of no enrichment, the double-gene knockout efficiency for TCR and B2M is up to 80% or more (The cells in the lower left quadrant of Figure 3D are CD3-negative and B2M-negative cells (double negative cells), representing the cells with double-gene knockout). Among the published materials, there is no method that can achieve such high double-gene knockout efficiency, which indicates that our method has great application potential in the efficient preparation of gene-edited primary cells suitable for allogeneic transplantation.

## Claims

1. A CRISPR-Cas system comprising guide RNAs and a Cas protein, wherein the guide RNAs are composed of the following two parts:
(a) a first guide RNA, capable of binding to a target sequence having SEQ ID NO: 1 in eukaryotic TRAC genes; and
(b) second and third guide RNAs, capable of respectively binding to two sequences of target sequences having SEQ ID NO: 2, SEQ ID NO: 3 and SEQ ID NO: 4 in eukaryotic B2M genes.

2. The CRISPR-Cas system of claim 1, wherein the second and third guide RNAs are capable of respectively binding to the target sequences having SEQ ID NO: 2 and SEQ ID NO: 3 in B2M genes, or binding to the target sequences having SEQ ID NO: 2 and SEQ ID NO: 4 or binding to the target sequences having SEQ ID NO: 3 and SEQ ID NO: 4.

3. The CRISPR-Cas system of claim 1 or 2, wherein the guide RNA is a single-stranded guide RNA.

4. The CRISPR-Cas system of claim 3, wherein the sequence of the first single-stranded guide RNA comprises a specific sequence 5'-GUCUCUCAGCUGGUACA-3'.

5. The CRISPR-Cas system of claim 4, wherein the sequence of the first single-stranded guide RNA is 5'-(X)n-GUCUCUCAGCUGGUACA-backbone sequence-3', in which X is any base selected from A, U, C and G, and n is any integer from 0 to 15, preferably n = 13, and more preferably n = 2.

6. The CRISPR-Cas system of claim 5, wherein the sequence of the first single-stranded guide RNA is SEQ ID NO: 8 or SEQ ID NO: 14.

7. The CRISPR-Cas system of any one of claims 1-6, wherein the sequence of the single-stranded guide RNA that binds to the target sequence of SEQ ID NO: 2 comprises a specific sequence 5'-cacgcuggauagccucc-3'; the sequence of the single-stranded guide RNA that binds to the target sequence of SEQ ID NO: 3 comprises a specific sequence 5'-uagcgcgagcacagcua-3'; and the sequence of the single-stranded guide RNA that binds to the target sequence of SEQ ID NO: 4 comprises a specific sequence 5'-cacggagcgagacaucu-3'.

8. The CRISPR-Cas system of claim 7, wherein the sequence of the single-stranded guide RNA that binds to the target sequence of SEQ ID NO: 2 is 5' (X)n-cacgcuggauagccucc-backbone sequence-3', the sequence of the single-stranded guide RNA that binds to the target sequence of SEQ ID NO: 3 is 5' (X)n-uagcgcgagcacagcua-backbone sequence-3', and the sequence of the single-stranded guide RNA that binds to the target sequence of SEQ ID NO: 4 is 5'-(X)n-cacggagcgagacaucu-backbone sequence-3', in which X is any base selected from A, U, C and G, and n is any integer from 0 to 15, preferably n = 13, and more preferably n = 2.

9. The CRISPR-Cas system of claim 8, wherein the sequence of the single-stranded guide RNA that binds to the target sequence of SEQ ID NO: 2 is SEQ ID NO: 15; the sequence of the single-stranded guide RNA that binds to the target sequence of SEQ ID NO: 3 is SEQ ID NO: 17; and the sequence of the single-stranded guide RNA that binds to the target sequence of SEQ ID NO: 4 is SEQ ID NO: 19.

10. The CRISPR-Cas system of claim 1 or 2, wherein the guide RNA is a double-stranded guide RNA comprising a crRNA and a tracrRNA.

11. The CRISPR-Cas system of claim 10, wherein the crRNA in the sequence of the first double-stranded guide RNA comprises a specific sequence 5'-GUCUCUCAGCUGGUACA-3'.

12. The CRISPR-Cas system of claim 11, wherein the crRNA in the sequence of the first double-stranded guide RNA is 5'-(X)n-GUCUCUCAGCUGGUACA-truncated backbone sequence-3', in which X is any base selected from A, U, C and G, and n is any integer from 0 to 15, preferably n = 13, and more preferably n = 2.

13. The CRISPR-Cas system of claim 11, wherein the crRNA in the sequence of the double-stranded guide RNA that binds to the target sequence of SEQ ID NO: 2 comprises a specific sequence 5'-cacgcuggauagccucc-3'; the crRNA in the sequence of the double-stranded guide RNA that binds to the target sequence of SEQ ID NO: 3 comprises a specific sequence 5'-uagcgcgagcacagcua-3'; and the crRNA in the sequence of the double-stranded guide RNA that binds to the target sequence of SEQ ID NO: 4 comprises a specific sequence 5'-cacggagcgagacaucu-3'.

14. The CRISPR-Cas system of claim 13, wherein the crRNA in the sequence of the double-stranded guide RNA that binds to the target sequence of SEQ ID NO: 2 comprises 5' (X)n-cacgcuggauagccucc-truncated backbone sequence-3', the crRNA in the sequence of the double-stranded guide RNA that binds to the target sequence of SEQ ID NO: 3 comprises 5' (X)n-uagcgcgagcacagcua-truncated backbone sequence-3', and the crRNA in the sequence of the double-stranded guide RNA that binds to the target sequence of SEQ ID NO: 4 comprises 5'-(X)n-cacggagcgagacaucu-truncated backbone sequence-3', in which X is any base selected from A, U, C and G, and n is any integer from 0 to 15, preferably n = 13, and more preferably n = 2.

15. The CRISPR-Cas system of claim 14, wherein the crRNA in the sequence of the first double-stranded guide RNA is SEQ ID NO: 25, and the tracrRNA in the sequence of the first double-stranded guide RNA is SEQ ID NO: 24.

16. The CRISPR-Cas system of any one of claims 1-15, wherein the Cas protein is a Cas9 protein, preferably a Cas9 protein derived from *Streptococcus pyogenes,* and more preferably a protein as shown in SEQ ID NO: 6.

17. The CRISPR-Cas system of any one of claims 1-16, wherein the guide RNA is chemically modified, preferably by means of modification with 2'-methoxy and 3'-phosphorothioate.

18. A CRISPR-Cas system comprising guide RNAs and a Cas protein, wherein the guide RNAs are composed of the following two parts: the guide RNAs are capable of respectively binding to two sequences of target sequences having SEQ ID NO: 2, SEQ ID NO: 3 and SEQ ID NO: 4 in eukaryotic B2M genes.

19. A method for preparing engineered eukaryotic cells, the method comprising transfecting the eukaryotic cells with the CRISPR-Cas system of any one of claims 1-18, and selectively inactivating genes encoding TRAC and B2M.

20. The method of claim 19, wherein the eukaryotic cells are lymphocytes, T cells, or peripheral blood mononuclear cells.

21. The method of claim 20, wherein the lymphocytes are mixed lymphocytes derived from different donor sources.


SEQUENCE LISTING

<110>  Cure Genetics CO., LTD

<120>  Gene Editing System and Gene Editing Method

<130>  AJ180030PCT

<160>  65

<170>  PatentIn version 3.5

<210>  1
<211>  19
<212>  DNA
<213>  Homo sapiens

<400>  1
gagtctctca gctggtaca                                                    19

<210>  2
<211>  19
<212>  DNA
<213>  Homo sapiens

<400>  2
ctcacgctgg atagcctcc                                                    19

<210>  3
<211>  19
<212>  DNA
<213>  Homo sapiens

<400>  3
agtagcgcga gcacagcta                                                    19

<210>  4
<211>  19
<212>  DNA
<213>  Homo sapiens

<400>  4
gccacggagc gagacatct                                                    19

<210>  5
<211>  19
<212>  DNA
<213>  Homo sapiens

<400>  5
aagttgactt actgaagaa                                                    19

<210>  6
<211>  1400
<212>  PRT
<213>  Streptococcus pyogenes

<400>  6

Met Ala Pro Lys Lys Lys Arg Lys Val Gly Ile His Gly Val Pro Ala
1               5                   10                  15

Ala Asp Lys Lys Tyr Ser Ile Gly Leu Asp Ile Gly Thr Asn Ser Val
            20                  25                  30

Gly Trp Ala Val Ile Thr Asp Glu Tyr Lys Val Pro Ser Lys Lys Phe
        35                  40                  45

Lys Val Leu Gly Asn Thr Asp Arg His Ser Ile Lys Lys Asn Leu Ile
    50                  55                  60

Gly Ala Leu Leu Phe Asp Ser Gly Glu Thr Ala Glu Ala Thr Arg Leu
65                  70                  75                  80

Lys Arg Thr Ala Arg Arg Arg Tyr Thr Arg Arg Lys Asn Arg Ile Cys
                85                  90                  95

Tyr Leu Gln Glu Ile Phe Ser Asn Glu Met Ala Lys Val Asp Asp Ser
            100                 105                 110

Phe Phe His Arg Leu Glu Glu Ser Phe Leu Val Glu Glu Asp Lys Lys
        115                 120                 125

His Glu Arg His Pro Ile Phe Gly Asn Ile Val Asp Glu Val Ala Tyr
    130                 135                 140

His Glu Lys Tyr Pro Thr Ile Tyr His Leu Arg Lys Lys Leu Val Asp
145                 150                 155                 160

Ser Thr Asp Lys Ala Asp Leu Arg Leu Ile Tyr Leu Ala Leu Ala His
                165                 170                 175

Met Ile Lys Phe Arg Gly His Phe Leu Ile Glu Gly Asp Leu Asn Pro
            180                 185                 190

Asp Asn Ser Asp Val Asp Lys Leu Phe Ile Gln Leu Val Gln Thr Tyr
        195                 200                 205

Asn Gln Leu Phe Glu Glu Asn Pro Ile Asn Ala Ser Gly Val Asp Ala
    210                 215                 220

Lys Ala Ile Leu Ser Ala Arg Leu Ser Lys Ser Arg Arg Leu Glu Asn
225                 230                 235                 240

Leu Ile Ala Gln Leu Pro Gly Glu Lys Lys Asn Gly Leu Phe Gly Asn
                245                 250                 255

Leu Ile Ala Leu Ser Leu Gly Leu Thr Pro Asn Phe Lys Ser Asn Phe
            260                 265                 270

Asp Leu Ala Glu Asp Ala Lys Leu Gln Leu Ser Lys Asp Thr Tyr Asp
        275                 280                 285

Asp Asp Leu Asp Asn Leu Leu Ala Gln Ile Gly Asp Gln Tyr Ala Asp
    290                 295                 300

Leu Phe Leu Ala Ala Lys Asn Leu Ser Asp Ala Ile Leu Leu Ser Asp
305                 310                 315                 320

Ile Leu Arg Val Asn Thr Glu Ile Thr Lys Ala Pro Leu Ser Ala Ser
                325                 330                 335

Met Ile Lys Arg Tyr Asp Glu His His Gln Asp Leu Thr Leu Leu Lys
            340                 345                 350

Ala Leu Val Arg Gln Gln Leu Pro Glu Lys Tyr Lys Glu Ile Phe Phe
        355                 360                 365

Asp Gln Ser Lys Asn Gly Tyr Ala Gly Tyr Ile Asp Gly Gly Ala Ser
    370                 375                 380

Gln Glu Glu Phe Tyr Lys Phe Ile Lys Pro Ile Leu Glu Lys Met Asp
385                 390                 395                 400

Gly Thr Glu Glu Leu Leu Val Lys Leu Asn Arg Glu Asp Leu Leu Arg
                405                 410                 415

Lys Gln Arg Thr Phe Asp Asn Gly Ser Ile Pro His Gln Ile His Leu
            420                 425                 430

Gly Glu Leu His Ala Ile Leu Arg Arg Gln Glu Asp Phe Tyr Pro Phe
        435                 440                 445

Leu Lys Asp Asn Arg Glu Lys Ile Glu Lys Ile Leu Thr Phe Arg Ile
    450                 455                 460

Pro Tyr Tyr Val Gly Pro Leu Ala Arg Gly Asn Ser Arg Phe Ala Trp
465                 470                 475                 480

Met Thr Arg Lys Ser Glu Glu Thr Ile Thr Pro Trp Asn Phe Glu Glu
                485                 490                 495

Val Val Asp Lys Gly Ala Ser Ala Gln Ser Phe Ile Glu Arg Met Thr

            500                 505                 510

Asn Phe Asp Lys Asn Leu Pro Asn Glu Lys Val Leu Pro Lys His Ser
        515                 520                 525

Leu Leu Tyr Glu Tyr Phe Thr Val Tyr Asn Glu Leu Thr Lys Val Lys
    530                 535                 540

Tyr Val Thr Glu Gly Met Arg Lys Pro Ala Phe Leu Ser Gly Glu Gln
545                 550                 555                 560

Lys Lys Ala Ile Val Asp Leu Leu Phe Lys Thr Asn Arg Lys Val Thr
                565                 570                 575

Val Lys Gln Leu Lys Glu Asp Tyr Phe Lys Lys Ile Glu Cys Phe Asp
            580                 585                 590

Ser Val Glu Ile Ser Gly Val Glu Asp Arg Phe Asn Ala Ser Leu Gly
        595                 600                 605

Thr Tyr His Asp Leu Leu Lys Ile Ile Lys Asp Lys Asp Phe Leu Asp
    610                 615                 620

Asn Glu Glu Asn Glu Asp Ile Leu Glu Asp Ile Val Leu Thr Leu Thr
625                 630                 635                 640

Leu Phe Glu Asp Arg Glu Met Ile Glu Glu Arg Leu Lys Thr Tyr Ala
                645                 650                 655

His Leu Phe Asp Asp Lys Val Met Lys Gln Leu Lys Arg Arg Arg Tyr
            660                 665                 670

Thr Gly Trp Gly Arg Leu Ser Arg Lys Leu Ile Asn Gly Ile Arg Asp
        675                 680                 685

Lys Gln Ser Gly Lys Thr Ile Leu Asp Phe Leu Lys Ser Asp Gly Phe
    690                 695                 700

Ala Asn Arg Asn Phe Met Gln Leu Ile His Asp Asp Ser Leu Thr Phe
705                 710                 715                 720

Lys Glu Asp Ile Gln Lys Ala Gln Val Ser Gly Gln Gly Asp Ser Leu
                725                 730                 735

His Glu His Ile Ala Asn Leu Ala Gly Ser Pro Ala Ile Lys Lys Gly
            740                 745                 750

Ile Leu Gln Thr Val Lys Val Val Asp Glu Leu Val Lys Val Met Gly
        755                 760                 765

Arg His Lys Pro Glu Asn Ile Val Ile Glu Met Ala Arg Glu Asn Gln
    770                 775                 780

Thr Thr Gln Lys Gly Gln Lys Asn Ser Arg Glu Arg Met Lys Arg Ile
785                 790                 795                 800

Glu Glu Gly Ile Lys Glu Leu Gly Ser Gln Ile Leu Lys Glu His Pro
                805                 810                 815

Val Glu Asn Thr Gln Leu Gln Asn Glu Lys Leu Tyr Leu Tyr Tyr Leu
            820                 825                 830

Gln Asn Gly Arg Asp Met Tyr Val Asp Gln Glu Leu Asp Ile Asn Arg
        835                 840                 845

Leu Ser Asp Tyr Asp Val Asp His Ile Val Pro Gln Ser Phe Leu Lys
    850                 855                 860

Asp Asp Ser Ile Asp Asn Lys Val Leu Thr Arg Ser Asp Lys Asn Arg
865                 870                 875                 880

Gly Lys Ser Asp Asn Val Pro Ser Glu Glu Val Val Lys Lys Met Lys
                885                 890                 895

Asn Tyr Trp Arg Gln Leu Leu Asn Ala Lys Leu Ile Thr Gln Arg Lys
            900                 905                 910

Phe Asp Asn Leu Thr Lys Ala Glu Arg Gly Gly Leu Ser Glu Leu Asp
        915                 920                 925

Lys Ala Gly Phe Ile Lys Arg Gln Leu Val Glu Thr Arg Gln Ile Thr
    930                 935                 940

Lys His Val Ala Gln Ile Leu Asp Ser Arg Met Asn Thr Lys Tyr Asp
945                 950                 955                 960

Glu Asn Asp Lys Leu Ile Arg Glu Val Lys Val Ile Thr Leu Lys Ser
                965                 970                 975

Lys Leu Val Ser Asp Phe Arg Lys Asp Phe Gln Phe Tyr Lys Val Arg
            980                 985                 990

Glu Ile Asn Asn Tyr His His Ala His Asp Ala Tyr Leu Asn Ala Val
        995                 1000                1005

Val Gly Thr Ala Leu Ile Lys Lys Tyr Pro Lys Leu Glu Ser Glu
    1010                1015                1020

Phe Val Tyr Gly Asp Tyr Lys Val Tyr Asp Val Arg Lys Met Ile
    1025                1030                1035

Ala Lys Ser Glu Gln Glu Ile Gly Lys Ala Thr Ala Lys Tyr Phe
    1040                1045                1050

Phe Tyr Ser Asn Ile Met Asn Phe Phe Lys Thr Glu Ile Thr Leu
    1055                1060                1065

Ala Asn Gly Glu Ile Arg Lys Arg Pro Leu Ile Glu Thr Asn Gly
    1070                1075                1080

Glu Thr Gly Glu Ile Val Trp Asp Lys Gly Arg Asp Phe Ala Thr
    1085                1090                1095

Val Arg Lys Val Leu Ser Met Pro Gln Val Asn Ile Val Lys Lys
    1100                1105                1110

Thr Glu Val Gln Thr Gly Gly Phe Ser Lys Glu Ser Ile Leu Pro
    1115                1120                1125

Lys Arg Asn Ser Asp Lys Leu Ile Ala Arg Lys Lys Asp Trp Asp
    1130                1135                1140

Pro Lys Lys Tyr Gly Gly Phe Asp Ser Pro Thr Val Ala Tyr Ser
    1145                1150                1155

Val Leu Val Val Ala Lys Val Glu Lys Gly Lys Ser Lys Lys Leu
    1160                1165                1170

Lys Ser Val Lys Glu Leu Leu Gly Ile Thr Ile Met Glu Arg Ser
    1175                1180                1185

Ser Phe Glu Lys Asn Pro Ile Asp Phe Leu Glu Ala Lys Gly Tyr
    1190                1195                1200

Lys Glu Val Lys Lys Asp Leu Ile Ile Lys Leu Pro Lys Tyr Ser
    1205                1210                1215

Leu Phe Glu Leu Glu Asn Gly Arg Lys Arg Met Leu Ala Ser Ala
    1220                1225                1230

Gly Glu Leu Gln Lys Gly Asn Glu Leu Ala Leu Pro Ser Lys Tyr
    1235                1240                1245

Val Asn Phe Leu Tyr Leu Ala Ser His Tyr Glu Lys Leu Lys Gly
    1250                1255                1260

Ser Pro Glu Asp Asn Glu Gln Lys Gln Leu Phe Val Glu Gln His
    1265                1270                1275

Lys His Tyr Leu Asp Glu Ile Ile Glu Gln Ile Ser Glu Phe Ser
    1280                1285                1290

Lys Arg Val Ile Leu Ala Asp Ala Asn Leu Asp Lys Val Leu Ser
    1295                1300                1305

Ala Tyr Asn Lys His Arg Asp Lys Pro Ile Arg Glu Gln Ala Glu
    1310                1315                1320

Asn Ile Ile His Leu Phe Thr Leu Thr Asn Leu Gly Ala Pro Ala
    1325                1330                1335

Ala Phe Lys Tyr Phe Asp Thr Thr Ile Asp Arg Lys Arg Tyr Thr
    1340                1345                1350

Ser Thr Lys Glu Val Leu Asp Ala Thr Leu Ile His Gln Ser Ile
    1355                1360                1365

Thr Gly Leu Tyr Glu Thr Arg Ile Asp Leu Ser Gln Leu Gly Gly
    1370                1375                1380

Asp Lys Arg Pro Ala Ala Thr Lys Lys Ala Gly Gln Ala Lys Lys
    1385                1390                1395

Lys Lys
    1400

<210>  7
<211>  80
<212>  RNA
<213>  Synthetic Sequence

<400>  7
guuuuagagc uagaaauagc aaguuaaaau aaggcuaguc cguuaucaac uugaaaaagu      60

ggcaccgagu cggugcuuuu                                                  80

<210>  8
<211>  99
<212>  RNA
<213>  Synthetic Sequence

<400>  8

gagucucuca gcugguacag uuuuagagcu agaaauagca aguuaaaaua aggcuagucc 60

guuaucaacu ugaaaaagug gcaccgaguc ggugcuuuu 99

<210> 9
<211> 90
<212> RNA
<213> Synthetic Sequence

<400> 9
guuuuagagc uaugcuggaa acagcauagc aaguuaaaau aaggcuaguc cguuaucaac 60

uugaaaaagu ggcaccgagu cggugcuuuu 90

<210> 10
<211> 17
<212> DNA
<213> Synthetic Sequence

<400> 10
gttttagagc tatgctg 17

<210> 11
<211> 90
<212> DNA
<213> Synthetic Sequence

<400> 11
aaaagcaccg actcggtgcc actttttcaa gttgataacg gactagcctt attttaactt 60

gctatgctgt ttccagcata gctctaaaac 90

<210> 12
<211> 55
<212> DNA
<213> Synthetic Sequence

<400> 12
taatacgact cactataggg agtctctcag ctggtacagt tttagagcta tgctg 55

<210> 13
<211> 128
<212> DNA
<213> Synthetic Sequence

<400> 13
taatacgact cactataggg agtctctcag ctggtacagt tttagagcta tgctggaaac 60

agcatagcaa gttaaaataa ggctagtccg ttatcaactt gaaaaagtgg caccgagtcg 120

gtgctttt 128

<210> 14
<211> 109
<212> RNA
<213> Synthetic Sequence

<400>  14
gagucucuca gcugguacag uuuuagagcu augcuggaaa cagcauagca aguuaaaaua      60

aggcuagucc guuaucaacu ugaaaaagug gcaccgaguc ggugcuuuu                 109


<210>  15
<211>  109
<212>  RNA
<213>  Synthetic Sequence

<400>  15
cucacgcugg auagccuccg uuuuagagcu augcuggaaa cagcauagca aguuaaaaua      60

aggcuagucc guuaucaacu ugaaaaagug gcaccgaguc ggugcuuuu                 109


<210>  16
<211>  55
<212>  DNA
<213>  Synthetic Sequence

<400>  16
taatacgact cactataggc tcacgctgga tagcctccgt tttagagcta tgctg           55


<210>  17
<211>  109
<212>  RNA
<213>  Synthetic Sequence

<400>  17
aguagcgcga gcacagcuag uuuuagagcu augcuggaaa cagcauagca aguuaaaaua      60

aggcuagucc guuaucaacu ugaaaaagug gcaccgaguc ggugcuuuu                 109


<210>  18
<211>  55
<212>  DNA
<213>  Synthetic Sequence

<400>  18
taatacgact cactatagga gtagcgcgag cacagctagt tttagagcta tgctg           55


<210>  19
<211>  109
<212>  RNA
<213>  Synthetic Sequence

<400>  19
gccacggagc gagacaucug uuuuagagcu augcuggaaa cagcauagca aguuaaaaua      60

aggcuagucc guuaucaacu ugaaaaagug gcaccgaguc ggugcuuuu                 109


<210>  20
<211>  55
<212>  DNA
<213>  Synthetic Sequence

<400>  20

taatacgact cactataggg ccacggagcg agacatctgt tttagagcta tgctg 55

<210> 21
<211> 109
<212> RNA
<213> Synthetic Sequence

<400> 21
aaguugacuu acugaagaag uuuuagagcu augcuggaaa cagcauagca aguuaaaaua 60

aggcuagucc guuaucaacu ugaaaaagug gcaccgaguc ggugcuuuu 109

<210> 22
<211> 55
<212> DNA
<213> Synthetic Sequence

<400> 22
taatacgact cactatagga agttgactta ctgaagaagt tttagagcta tgctg 55

<210> 23
<211> 16
<212> RNA
<213> Synthetic Sequence

<400> 23
guuuuagagc uaugcu 16

<210> 24
<211> 67
<212> RNA
<213> Streptococcus pyogenes

<400> 24
agcauagcaa guuaaaauaa ggcuaguccg uuaucaacuu gaaaaagugg caccgagucg 60

gugcuuu 67

<210> 25
<211> 35
<212> RNA
<213> Synthetic Sequence

<400> 25
gagucucuca gcugguacag uuuuagagcu augcu 35

<210> 26
<211> 82
<212> RNA
<213> Synthetic Sequence

<400> 26
guuuuagagc uagaaauagc aaguuaaaau aaggcuaguc cguuaucaac uugaaaaagu 60

ggcaccgagu cggugcuuuu uu 82

<210>  27
<211>  50
<212>  RNA
<213>  Synthetic Sequence

<400>  27
guuuuagagc uagaaauagc aaguuaaaau aaggcuaguc cguuuuuuuu                50


<210>  28
<211>  56
<212>  RNA
<213>  Synthetic Sequence

<400>  28
guuuuagagc uagaaauagc aaguuaaaau aaggcuaguc cguuaucauu uuuuuu          56


<210>  29
<211>  69
<212>  RNA
<213>  Synthetic Sequence

<400>  29
guuuuagagc uagaaauagc aaguuaaaau aaggcuaguc cguuaucaac uugaaaaagu      60

guuuuuuuu                                                              69


<210>  30
<211>  92
<212>  RNA
<213>  Synthetic Sequence

<400>  30
guuuuagagc uaugcuggaa acagcauagc aaguuaaaau aaggcuaguc cguuaucaac      60

uugaaaaagu ggcaccgagu cggugcuuuu uu                                    92


<210>  31
<211>  92
<212>  RNA
<213>  Synthetic Sequence

<400>  31
guuucagagc uaugcuggaa acagcauagc aaguugaaau aaggcuaguc cguuaucaac      60

uugaaaaagu ggcaccgagu cggugcuuuu uu                                    92


<210>  32
<211>  84
<212>  RNA
<213>  Synthetic Sequence

<400>  32
guuuuagagc uaugaaaaua gcaaguuaaa auaaggcuag uccguuauca acuugaaaaa      60

guggcaccga gucggugcuu uuuu                                             84


<210>  33

<211>  88
<212>  RNA
<213>  Synthetic Sequence

<400>  33
guuuuagagc uaugcgaaag cauagcaagu uaaaauaagg cuaguccguu aucaacuuga      60

aaaaguggca ccgagucggu gcuuuuuu                                          88


<210>  34
<211>  98
<212>  RNA
<213>  Synthetic Sequence

<400>  34
guuuuagagc uaugcuguuu gaaaaaacag cauagcaagu uaaaauaagg cuaguccguu      60

aucaacuuga aaaaguggca ccgagucggu gcuuuuuu                              98


<210>  35
<211>  102
<212>  RNA
<213>  Synthetic Sequence

<400>  35
guuuuagagc uaugcuguuu uggaaacaaa acagcauagc aaguuaaaau aaggcuaguc      60

cguuaucaac uugaaaaagu ggcaccgagu cggugcuuuu uu                        102


<210>  36
<211>  16
<212>  RNA
<213>  Streptococcus pyogenes

<400>  36
guuuuagagc uaugcu                                                       16


<210>  37
<211>  12
<212>  RNA
<213>  Streptococcus pyogenes

<400>  37
guuuuagagc ua                                                           12


<210>  38
<211>  17
<212>  RNA
<213>  Streptococcus pyogenes

<400>  38
guuuuagagc uaugcug                                                      17


<210>  39
<211>  17
<212>  RNA
<213>  Streptococcus pyogenes

<400>  39
guuucagagc uaugcug                                                    17


<210>  40
<211>  13
<212>  RNA
<213>  Streptococcus pyogenes

<400>  40
guuuuagagc uau                                                        13


<210>  41
<211>  14
<212>  RNA
<213>  Streptococcus pyogenes

<400>  41
guuuuagagc uaug                                                       14


<210>  42
<211>  15
<212>  RNA
<213>  Streptococcus pyogenes

<400>  42
guuuuagagc uaugc                                                      15


<210>  43
<211>  19
<212>  RNA
<213>  Streptococcus pyogenes

<400>  43
guuuuagagc uaugcuguu                                                  19


<210>  44
<211>  20
<212>  RNA
<213>  Streptococcus pyogenes

<400>  44
guuuuagagc uaugcuguuu                                                 20


<210>  45
<211>  22
<212>  RNA
<213>  Streptococcus pyogenes

<400>  45
guuuuagagc uaugcuguuu ug                                              22


<210>  46
<211>  67
<212>  RNA
<213>  Streptococcus pyogenes

<400>  46
agcauagcaa guuaaaauaa ggcuaguccg uuaucaacuu gaaaaagugg caccgagucg      60

gugcuuu                                                                67


<210>  47
<211>  66
<212>  RNA
<213>  Streptococcus pyogenes

<400>  47
uagcaaguua aaauaaggcu aguccguuau caacuugaaa aaguggcacc gagucggugc      60

uuuuuu                                                                 66


<210>  48
<211>  34
<212>  RNA
<213>  Streptococcus pyogenes

<400>  48
uagcaaguua aaauaaggcu aguccguuuu uuuu                                  34


<210>  49
<211>  40
<212>  RNA
<213>  Streptococcus pyogenes

<400>  49
uagcaaguua aaauaaggcu aguccguuau cauuuuuuuu                            40


<210>  50
<211>  53
<212>  RNA
<213>  Streptococcus pyogenes

<400>  50
uagcaaguua aaauaaggcu aguccguuau caacuugaaa aaguguuuuu uuu             53


<210>  51
<211>  71
<212>  RNA
<213>  Streptococcus pyogenes

<400>  51
cagcauagca aguuaaaaua aggcuagucc guuaucaacu ugaaaaagug gcaccgaguc      60

ggugcuuuuu u                                                           71


<210>  52
<211>  71
<212>  RNA
<213>  Streptococcus pyogenes

<400>  52
cagcauagca aguugaaaua aggcuagucc guuaucaacu ugaaaaagug gcaccgaguc      60

ggugcuuuuu u                                                          71

<210>  53
<211>  67
<212>  RNA
<213>  Streptococcus pyogenes

<400>  53
auagcaaguu aaaauaaggc uaguccguua ucaacuugaa aaaguggcac cgagucggug      60

cuuuuuu                                                              67

<210>  54
<211>  69
<212>  RNA
<213>  Streptococcus pyogenes

<400>  54
gcauagcaag uuaaaauaag gcuaguccgu uaucaacuug aaaaaguggc accgagucgg      60

ugcuuuuuu                                                            69

<210>  55
<211>  73
<212>  RNA
<213>  Streptococcus pyogenes

<400>  55
aacagcauag caaguuaaaa uaaggcuagu ccguuaucaa cuugaaaaag uggcaccgag      60

ucggugcuuu uuu                                                       73

<210>  56
<211>  74
<212>  RNA
<213>  Streptococcus pyogenes

<400>  56
aaacagcaua gcaaguuaaa auaaggcuag uccguuauca acuugaaaaa guggcaccga      60

gucggugcuu uuuu                                                      74

<210>  57
<211>  76
<212>  RNA
<213>  Streptococcus pyogenes

<400>  57
caaaacagca uagcaaguua aaauaaggcu aguccguuau caacuugaaa aaguggcacc      60

gagucggugc uuuuuu                                                    76

<210>  58
<211>  88
<212>  RNA
<213>  Streptococcus pyogenes

<400>  58
guuggaacca uucaaaacag cauagcaagu uaaaauaagg cuaguccguu aucaacuuga      60

aaaaguggca ccgagucggu gcuuuuuu                                          88



<210>  59
<211>  67
<212>  RNA
<213>  Streptococcus pyogenes

<400>  59
cauagcaagu uaaaauaagg cuaguccguu aucaacuuga aaaaguggca ccgagucggu      60

gcuuuuu                                                                 67



<210>  60
<211>  57
<212>  RNA
<213>  Streptococcus pyogenes

<400>  60
agcauagcaa guuaaaauag uuaucaacuu gaaaaagugg caccgagucg gugcuuu         57



<210>  61
<211>  51
<212>  RNA
<213>  Streptococcus pyogenes

<400>  61
agcauagcaa guuaaaauaa acuugaaaaa guggcaccga gucggugcuu u               51



<210>  62
<211>  63
<212>  RNA
<213>  Streptococcus pyogenes

<400>  62
agcauagcaa guuaaaauaa ggcuaguccg uuaucaacuu gaaaaagugg caccgagucg      60

gug                                                                     63



<210>  63
<211>  62
<212>  RNA
<213>  Streptococcus pyogenes

<400>  63
agcauagcaa guuaaaauaa ggcuaguccg uuaucaacuu gaaaaagugg caccgagucg      60

gu                                                                      62



<210>  64
<211>  61
<212>  RNA
<213>  Streptococcus pyogenes

<400>  64
agcauagcaa guuaaaauaa ggcuaguccg uuaucaacuu gaaaaagugg caccgagucg      60

g                                                                      61



<210>  65
<211>  58
<212>  RNA
<213>  Streptococcus pyogenes

<400>  65
agcauagcaa guuaaaauaa ggcuaguccg uuaucaacuu gaaaaagugc cgagucgg        58